(19) **Europäisches Patentamt**
**European Patent Office**
**Office européen des brevets**

(11) **EP 1 838 295 B1**

(12) **EUROPEAN PATENT SPECIFICATION**

(45) Date of publication and mention
of the grant of the patent:
**23.12.2009 Bulletin 2009/52**

(51) Int Cl.:
***A61K 31/00*** *(2006.01)*

(21) Application number: **06700797.1**

(22) Date of filing: **19.01.2006**

(86) International application number:
**PCT/GB2006/000211**

(87) International publication number:
**WO 2006/077429 (27.07.2006 Gazette 2006/30)**

(54) **ANTHELMINTIC COMPOSITION**

ANTHELMINTHISCHE ZUSAMMENSETZUNG

PREPARATION ANTHELMINTHIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.01.2005 GB 0501220**

(43) Date of publication of application:
**03.10.2007 Bulletin 2007/40**

(73) Proprietor: **NORBROOK LABORATORIES LIMITED**
**Newry, County Down BT35 6JP (GB)**

(72) Inventors:
• **BLAKELY, William**
**Newry,**
**County Down BT35 8JZ (GB)**

• **CROMIE, Lillian**
**Newry,**
**County Down BT34 2PN (GB)**

(74) Representative: **Hayes, Adrian Chetwynd et al**
**Boult Wade Tennant**
**Verulam Gardens**
**70 Gray's Inn Road**
**London WC1X 8BT (GB)**

(56) References cited:
**EP-A- 0 125 004        WO-A1-97/26895**
**US-A1- 2003 180 350**

Remarks:
The file contains technical information submitted after the application was filed and not included in this specification

## Description

Field of the Invention

This invention relates to a composition displaying efficacy against infections of Fasciola hepatica, Ostertagia ostertagi and Cooperia oncophora in susceptible ruminants, especially cattle.

Background of the Invention

Infections of Fasciola hepatica, Ostertagia ostertagi and Cooperia oncophora in livestock are problematic, and in particular first season grazing cattle can be susceptible in contaminated pastures. Injection solutions containing ivermectin and clorsulon are known in the industry for use in treating beef and non-lactating cattle.

Clorsulon is a compound belonging to the benzenesulphonamide (benzenesulfonamide -USA) family which is recommended for control of adult liver flukes (Fasciola hepatica and Fasciola gigantica) in cattle as suspensions for oral use or in injectable formulations for subcutaneous administrations. The oral recommended level is 7 mg/kg body weight (bw) and the subcutaneous level 2mg/kg bw. Currently, there is no known topical formulation, and this is likely to be due to foreseeable difficulties in achieving adequate penetration of the skin by clorsulon.

US-A-2003/0180350 discloses a veterinary composition for incorporating both hydrophilic and lipophilic drugs through the use of medium chain mono- and di-glycerdies.

EP-A-0 125 004 discloses as synergistic anti-parasite composition comprising clorsulon and ivermectin.

An objective of the, invention is to provide a topical formulation which is effective against the aforesaid infections, preferably presented as a pour-on formulation. A further object of the invention is provide a composition comprising at least one effective agent derived from Streptomyces avermitilis, i.e. a macrocyclic lactone or chemically modified or synthetic derivative thereof together with another anthelmintic of the sulphonamide type. Another objective of the invention is to provide a composition suitable for the treatment of immature *Fasciola hepatica.*

With this objective in mind, a study was conducted to determine the feasibility of producing a topical formulation containing known agents already recognised in the field in other delivery forms, e.g. injections, as offering efficacy' against infections of *Fasciola hepatica* (Adult), Ostertagia *ostertagi* (Adult) and *Cooperia oncophora* (Adult) in cattle.

The new formulation under consideration for the purposes of this study would include at least one anthelmintic compound of the disulphonamide type, e.g. clorsulon, a member of the benzenesulphonamide family (CAS.No. 60200-06-84; Amino-6-(trichloroethenyl)-1,3-benzene-disulfonamide), and an avermectin suitable for the treatment of immature *Fasciola hepatica* e.g. ivermectin, a mixture of semi-synthetic macrocyclic lactones (CAS.No. 70288-86-7; "22, 23-dihydro-C076B" a mixture of 80% ivermectin component $B_{1a}$(5-O-demethyl-22,23-dihydroavermectin $A_{1a}$) and 20% ivermectin component $B_{1b}$(5-O-demethyl-35-de(1-methylpropyl)-22, 23-dihydro-35-(1-methylethyl)avermectin $A_{1a}$)).

This study followed an authorised protocol in accordance with recognised industry practice, at an accredited animal facility ofNorbrook Laboratories Limited, Research Division, upon healthy male calves of European bovine stock.

During the study each animal was infected with 500 metacercariae of *Fasciola hepatica,* 10000 *Ostertagia ostertagi* larvae and 10000 *Cooperia oncophora* larvae. At 79 days following the administration of *Fasciola hepatica* and 32 days following administration of *Ostertagia ostertagi* and *Cooperia oncophora* the animals were treated with a pour-on formulation newly developed by Norbrook Laboratories Limited, comprising ivermectin and clorsulon. Approximately 3 weeks following treatment all animals were slaughtered and livers, abomasums and small intestines removed. These organs were processed to allow enumeration of helminths contained in each.

The calves used in the study were all healthy at selection and throughout the study period. The tested pour-on product was well tolerated in cattle and no adverse reactions to treatment were observed during the course of the study.

Enumeration of *Fasciola hepatica, Ostertagia ostertagi* and *Cooperia oncophora* burdens after slaughter and comparison to those of the untreated control group confirmed an efficacy of > 90% for each parasite and therefore it can be concluded that the tested pour-on product is effective in the treatment of adult *Fasciola hepatica,* adult *Ostertagia ostertagi* and adult *Cooperia oncophora* infections in cattle.

The efficacy of the tested pour-on product, in this instance comprising ivermectin and clorsulon, against induced infections of adult *Fasciola hepatica,* adult *Ostertagia ostertagi* and adult *Cooperia oncophora* cattle following topical administration at a nominal dose rate of 500 μg ivermectin and 5mg clorsulon per kg bodyweight was recognised.

Summary of the Invention

Accordingly, the present invention provides a hitherto unavailable topical formulation comprising as active ingredients, at least one effective agent derived from *Streptomyces avermitilis,* i.e. a macrocyclic lactone e.g. an avermectin or chemically modified or synthetic derivative thereof, e.g. ivermectin, together with another anthelmintic of the sulphonamide type, e.g. clorsulon, in a carrier that facilitates topical administration and delivery of the active ingredients transdermally. The carrier comprises alcoholic solvents, with optional excipients and formulation aids, which may comprise a polymeric species such as PVP or a poloxamer. In preferred form, a pour-on formulation is provided containing clorsulon and ivermectin. The alcoholic solvents comprise at least 30% (v/v) of ethanol, together with isopropanol to make the formulation up to 100%.

An embodiment of such a formulation of this invention is:

| | |
|---|---|
| Ivermectin 0.5% (w/v) | Clorsulon 5.0% (w/v) |
| Ethanol 30% v/v | PEG200 10% v/v |
| Crodamol Cap 20% v/v | IPA (isopropanol) to 100% v/v |
| Brilliant Blue Dye 0.01 %(w/v) | Denatonium Benzoate 0.001 %(w/v) |

The nominal dose rate thereof is 500 μg ivermectin and 5mg clorsulon per kg bodyweight.

A method of countering infection by immature *Fasciola hepatica* is enabled using such a formulation.

EXPERIMENTAL PROCEDURE

This study comprised a single group of four male calves, aged approximately 3 to 4 months old at the time of *Fasciola hepatica* administration. Faecal egg count examinations were carried out by an independent facility to determine that animals were helminth-free prior to infection with *Fasciola hepatica* metacercariae. The animals were acclimatized for 19 days prior to liver fluke administration.

Prior to treatment, (79 days pre treatment), all calves were initially infected with 500 metecercariae of *Fasciola hepatica* administered orally and subsequently, (32 days pre treatment) each calf was infected with 10000 larvae of *Ostertagia ostertagi* and 10000 larvae of *Cooperia oncophora* administered orally. The calves were weighed 3 days prior to administration of the subject pour-on product, to calculate the dose to be administered to each animal. The calves were slaughtered on the 18$^{th}$ day after treatment, with each animal's liver, abomasum and small intestine being removed and processed to allow helminth enumeration.

The pour-on product of the invention nominally contained 0.5% w/v ivermectin and 5.0% w/v clorsulon and had an assayed content of 0.498% w/v ivermectin and 4.94% w/v clorsulon. This provides a nominal dose rate of 500 μg ivermectin and 5mg clorsulon per kg bodyweight.

The pour-on product was administered by topical administration along the midline of the back on a narrow strip between the withers and the tail head on an area that was 24 inches long for each animal. This corresponds to the proposed route of administration for the pour-on product. All doses were administered to each animal on a single occasion using 20ml syringes which have an accuracy of 0.5ml. The doses administered to each animal are detailed in Table 1.

_Liver Fluke Count:_ Liver fluke were counted by emptying the contents if the labelled container into a flat-bottomed glass dish. A dark surface beneath the dish assisted in identifying small or immature flukes. A total count was recorded. Counting was carried out by an independent external service provider and was conducted blind with reference to group.

_Nematode Counts:_ The contents of each labelled jar were examined separately. Small quantities were poured into ruled Petri dishes with parallel lines marked I cm apart on their underside, and the worms were counted using a dissecting stereomicroscope. Since iodine had been added during processing of the samples to colour the nematodes, a solution of sodium thiosulphate was used to decolourise the background if necessary. The count for each jar was recorded separately, and multiplied by 100 (the original dilution factor). The series of 2 counts were averaged to give the final count. Nematodes were identified by picking out male worms into an embryo dish containing lactophenol and transferring onto microscope slides with a drop of lactophenol added and coverslips placed in top. The worms were identified using standard parasitological textbooks. Counting was carried out by an independent facility and was conducted blind with reference to group.

Analysis

After slaughter, the counts of *Fasciola hepatica, Ostertagia ostertagi* and *Cooperia oncophora* from each animal were enumerated and summarised using the mean, geometric mean, minimum, maximum and sample size. Since this study did not contain a control group, the control group results from a parallel study which utilised the same levels of infection with the infections at the same stage at slaughter (adult *Fasciola hepatica* and adult nematodes), were used to calculate efficacy as detailed below (See Tables 3a- 5b for all results).

$$\% \text{ efficacy} = \frac{\text{Geometric Mean of Controls} - \text{Geometric Mean Treated}}{\text{Geometric Mean of Controls}} \times 100$$

Group geometric means counts were compared by two sample t-test after logarithmic transformation to normalise the data. If the data did not follow the normal distribution then a non-parametric test was applied to compare the groups, names the Mann-Whitney test for unmatched pairs. No covariants were used in the data analysis.

RESULTS:

Faecal egg counts.

All faecal samples taken prior to treatment were found to be free of helminth eggs. Three of the four faecal samples taken 29 days after *Ostertagia ostertagi* and *Cooperia oncophora* infection, were found to contain strongyle-type eggs (See Table 2).

*Fasciola hepatica* counts after slaughter:

Summary of *Fasciola hepatica* counts.

|  | Treated | Control |
|---|---|---|
| Mean | 7.75 | 198.63 |
| Maximum | 19 | 264 |
| Minimum | 1 | 84 |
| Geometric mean | 5.16 | 189.38 |
| Number | 4 | 8 |

The overall mean establishment percentage of flukes in the untreated control group was 39.7%, this is slightly above the expected range (approximately 30-35% based on similar studies of this type) for the number of metacercariae administered (500 each) and age of cattle. The level of infection achieved is well above the minimal mean of 20 flukes per animal (as detailed in VICH GL 12 Efficacy of Anthelmintics: Specific Recommendations for Bovines) required to deem the infection adequate.

At the time of treatment, the *Fasciola hepatica* were within the 8 to 12 week range (as detailed in the VICH GL 12 Efficacy of Anthelmintics: Specific Requirements for Bovines) required to be classified as an adult.

The efficacy of flukicide treatment was 97.3% (See Table 6 for Summary of Efficacy Calculations). The statistical differences between the number of flukes recovered from treated test animals and untreated control animals were highly significant ($p=0.0117$) by the non-parametric Mann Whitney (Wilcoxon Rank-Sum) Test on log-transformed data, as the assumptions of normality were not satisfied. See Table 3 for individual *Fasciola hepatica* counts.

*Ostertagia ostertagi* counts after slaughter:

Summary of *Ostertagia ostertagi* counts.

|  | Treated | Control |
|---|---|---|
| Mean | 0 | 1937.50 |
| Maximum | 0 | 2950 |
| Minimum | 0 | 550 |
| Geometric mean | 0 | 1646.48 |
| Number | 4 | 8 |

The mean establishment percentage of *Ostertagia ostertagi* in the untreated control group was 19.4%, within the range expected (based on experience of similar studies) from the number of larvae administered (10000 each) and age of cattle. The level of infection achieved is well above the minimal mean of 100 nematodes per animal (as detailed in VICH GL 12 Efficacy of Anthelmintics: Specific Recommendations for Bovines) required to deem the infection adequate.

Treatment of test animals occurred on 32 days after administration of *Ostertagia ostertagi* larvae to all animals. Consequently, at the time of treatment the *Ostertagia ostertagi* were within the 28 to 32 day range (as detailed in VICH GL 12 Efficacy of Anthelmintics: Specific Recommendations for Bovines) required to be classified as adult.

The efficacy of treatment was 100.0% (See Table 6 for Summary for Efficacy Calculations). The statistical differences between the numbers of nematodes recovered from treated test animals and untreated control group animals were highly significant (p=0.0107) by the non-parametric Mann Whitney (Wilcoxon Rank-Sum) Test on log-transformed data, as the assumptions of normality were not satisfied. See Table 4 for individual *Ostertagia ostertagi* counts.

*Cooperia oncophora* counts after slaughter:

Summary of *Cooperia oncophora* counts.

|  | Treated | Control |
|---|---|---|
| Mean | 100.00 | 5662.50 |
| Maximum | 350 | 7950 |
| Minimum | 0 | 3750 |
| Geometric mean | 10.57 | 5485.59 |
| Number | 4 | 8 |

The establishment percentage from the untreated control group was 56.6%, within the range expected (based on experience of similar studies) from the number of larvae administered (10000 each) and age of cattle. The level of infection achieved is well above the minimal mean of 100 nematodes per animal (as detailed in VICH GL 12 Efficacy of Anthelmintics: Specific Recommendations for Bovines) required to deem the infection adequate.

At the time the *Cooperia oncophora* were within the 28 to 32 day range (as detailed in VICH GL 12 Efficacy of Anthelmintics: Specific Recommendations for Bovines) required to be classified as adult.

The efficacy of treatment was 99.8% (See Table 6 for Summary of Efficacy Calculations). The statistical differences between the numbers of nematodes recovered from treated test animals were highly significant (p=0.0244) by the parametric t test on log-transformed data, as the assumptions of normality were satisfied. See Table 5 for individual *Cooperia oncophora* counts.

From the results it has been demonstrated that administration of the pour-on product of this invention resulted in efficacy of 97.3% against *Fasciola hepatica* aged 11 weeks post-infection, and therefore meets the >90% efficacy requirement of VICH GL 12 (Efficacy of Anthelmintics: Specific Recommendations for Bovines). Data analysis has shown that the effect of treatment produced highly statistically significant differences in the fluke burden. The study also showed that the subject Ivermectin/Clorsulon Pour-On of the invention had an efficacy of 100.0% against adult *Ostertagia ostertagi* and 99.8% against adult *Cooperia ancophora,* again meeting the >90% Efficacy requirement of VICH GL 12. Data analysis has shown that the effect of treatment produced highly statistically significant differences in the nematode burden.

The pour-on product was well tolerated in cattle at application and no localised or systematic adverse reactions to treatment were recorded at any stage following treatment.

It can be concluded that the ivermectin/clorsulon pour-on product of this invention, administered topically at a dose rate of 500 μg ivermectin and 5mg clorsulon per kg bodyweight is clinically effective in the treatment of adult *Fasciola hepatica,* adult *Ostertagia ostertagi* and adult *Cooperia oncophora* in cattle. For each parasite efficacy was over 90% and there was a statistically significant difference(P<0.05) between the counts in treated and untreated animals. With this particular formulation blood plasma levels of clorsulon between 1.20 ug and 2.4ug per ml were observed in treated animals with Tmaxs of 54 +/- 22.9.

**Table 1. Animal and Dosage Details.**

| Animal Identity | Breed | Sex | Age[1] (months) | Weight[2] (kg) | Dose Administered (ml) |
|---|---|---|---|---|---|
| DY 1109-2 | Fr | M | 4.0 | 201.0 | 20.0 |
| DY 1819-7 | Fr | M | 3.5 | 154.0 | 15.5 |
| DY 1820-1 | Ayr | M | 4.0 | 141.0 | 14.0 |
| DY 2474-2 | Fr | M | 4.0 | 187.5 | 19.0 |

1 - Approximate age at selection.
2 -Animals weighed 3 days prior to administration of pour-on product.
**Ayr** = Ayrshire;
**Fr** = Friesian;
**M** = Male

**Table 2. Faecal Egg Counts (3 days prior to treatment)**

| Animal Identity | Strongyle Faecal Egg Count (epg) |
|---|---|
| DY 1109-2 | 0 |
| DY 1819-7 | 900 |
| DY 1820-1 | 700 |
| DY 2474-2 | 800 |

epg = eggs per gram.

**Table 3a. *Fasciola hepatica,* Counts (Treated).**

| Animal Identity | *Fasciola hepatica* Count |
|---|---|
| DY 1109-2 | 8 |
| DY 1819-7 | 1 |
| DY 2474-2 | 9 |
| | |
| Mean | 7.75 |
| Geometric Mean | 5.16 |
| Minimum | 1 |
| Maximum | 19 |
| Sample Size | 4 |

**Table 3b. *Fasciola hepatica* Counts (Control).**

| Animal Identity | *Fasciola hepatica* Count |
|---|---|
| DY 351-1 | 214 |
| DY 1097-3 | 165 |
| DY 1167-7 | 84 |
| DY 1184-3 | 257 |
| DY 1498-7 | 196 |
| DY 1499-1 | 264 |
| DY 1500-2 | 203 |

(continued)

| Animal Identity | *Fasciola hepatica* Count |
|---|---|
| DY 1577-7 | 206 |
| | |
| Mean | 198.63 |
| Geometric Mean | 189.38 |
| Minimum | 84 |
| Maximum | 264 |
| Sample Size | 8 |

**Table 4a.** *Ostertagia ostertagi* **Counts (Treated).**

| Animal Identity | *Ostertagia ostertagi* Count |
|---|---|
| DY 1109-2 | 0 |
| DY 1819-7 | 0 |
| DY 1820-1 | 0 |
| DY 2474-2 | 0 |
| | |
| Mean | 0 |
| Geometric Mean | 0 |
| Minimum | 0 |
| Maximum | 0 |
| Sample Size | 4 |

**Table 4b.** *Ostertagia ostertagi* **Counts,(Control).**

| Animal Identity | *Ostertagia ostertagi* Count |
|---|---|
| DY 351-1 | 2950 |
| DY 1097-3 | 550 |
| DY 1167-7 | 2650 |
| DY 1184-3 | 2550 |
| DY 1498-7 | 1800 |
| DY 1499-1 | 650 |
| DY 1500-2 | 2900 |
| DY 1577-7 | 1450 |
| | |
| Mean | 1937.50 |
| Geometric Mean | 1646.48 |
| Minimum | 550 |
| Maximum | 2950 |
| Sample Size | 8 |

**Table 5. *Cooperia oncophora* Counts (Treated).**

| Animal Identity | Count |
|---|---|
| DY 1109-2 | 0 |
| DY 1819-7 | 350 |
| DY1820-1 | 50 |
| DY 2474-2 | 0 |
| | |
| Mean | 100.00 |
| Geometric Mean | 10.57 |
| Minimum | 0 |
| Maximum | 350 |
| Sample Size | 4 |

**Table 5. *Cooperia oncophora* Counts (Control).**

| Animal Identity | *Fasciola hepatica* Count |
|---|---|
| DY 351-1 | 4800 |
| DY 1097-3 | 7950 |
| DY 1167-7 | 7900 |
| DY 1184-3 | 5550 |
| DY 1498-7 | 6050 |
| DY 1499-1 | 4800 |
| DY 1500-2 | 3750 |
| DY 1577-7 | 4500 |
| | |
| Mean | 5662.50 |
| Geometric Mean | 5485.59 |
| Minimum | 3750 |
| Maximum | 7950 |
| Sample Size | 8 |

**Table 6. Summary of Efficacy Calculations.**

| Helminth | Treated Geometric Mean | Control Group Geometric Mean | Percentage Efficacy |
|---|---|---|---|
| *Fasciola hepatica* | 5.16 | 189.38 | 97.3 |
| *Ostertagia ostertagi* | 0 | 1646.48 | 100.0 |
| *Cooperia oncophora* | 10.57 | 5485.59 | 99.8 |

**Claims**

1.  A topical anthelmintic formulation comprising as active ingredients, a therapeutically effective amount of at least one anthelmintic agent derived from *Streptomyces avermitilis,* with a therapeutically effective amount of at least one other anthelmintic of the sulphonamide type, in an alcoholic solvent-based carrier suitable for topical administration and delivery of the active ingredients transdermally, said carrier comprising at least 30% (v/v) of ethanol together with isopropanol quantity sufficient to 100% and optionally excipients and formulation aids.

2. A topical anthelmintic formulation according to claim 1, wherein the formulation is presented as a pour-on.

3. A topical anthelmintic formulation according to claim 1, wherein the formulation comprises an avermectin.

4. A topical anthelmintic formulation according to claim 1, wherein the formulation comprises ivermectin.

5. A topical anthelmintic formulation according to any one of the preceding claims, wherein the anthelmintic of the sulphonamide type is clorsulon.

6. A topical anthelmintic formulation according to claim 1 or claim 2, comprising clorsulon and ivermectin.

7. A topical anthelmintic formulation according to any one of the preceding claims, having an efficacy such that, when applied to the skin of an animal infected by *F. hepatica,* at least 90% of the mature *F. hepatica* are killed.

8. A topical anthelmintic formulation according to claim 5, wherein the therapeutically effective amount for cattle of clorsulon is at least 5% (w/v).

9. A topical anthelmintic formulation according to any one of the preceding claims, wherein the carrier comprises a polymeric species.

10. A topical anthelmintic formulation according to claim 1, which in use provides at least 2 $\mu$g of a benzenesulphonamide per ml of blood plasma.

11. A topical anthelmintic formulation according to claim 6 for use on cattle, wherein the dosage rate is controlled to provide about 500 $\mu$g ivermectin and 5mg/kg of clorsulon.

12. A topical anthelmintic formulation for use on cattle, presented as a pour-on and consisting of:

| | |
|---|---|
| Ivermectin 0.5% (w/v) | Clorsulon 5.0% (w/v) |
| Ethanol 30% v/v | PEG200 10% v/v |
| Crodamol Cap 20% v/v | IPA (isopropanol) to 100% v/v |
| Brilliant Blue Dye 0.01%(w/v) | Denatonium Benzoate 0.001 %(w/v) |

## Patentansprüche

1. Topische anthelmintische Formulierung, umfassend als Wirkstoffe eine therapeutisch wirksame Menge mindestens eines anthelmintischen Mittels, das von *Streptomyces avermitilis* stammt, mit einer therapeutisch wirksamen Menge mindestens eines anderen Anthelmintikums des Sulfonamidtyps, in einem Lösungsmittelträger auf Alkoholbasis, der geeignet ist für topische Anwendung und transdermale Zulieferung der Wirkstoffe, wobei der Träger mindestens 30 % (VN) Ethanol zusammen mit einer Isopropanolmenge umfasst, um 100 % zu bilden, und optional Exzipienten und Formulierungshilfsmittel umfasst.

2. Topische anthelmintische Formulierung nach Anspruch 1, worin die Formulierung als eine Aufgießformulierung vorliegt.

3. Topische anthelmintische Formulierung nach Anspruch 1, worin die Formulierung ein Avermectin umfasst.

4. Topische anthelmintische Formulierung nach Anspruch 1, worin die Formulierung ein Ivermectin umfasst.

5. Topische anthelmintische Formulierung nach einem der vorhergehenden Ansprüche, worin das Anthelmintikum des Sulfonamidtyps Clorsulon ist.

**6.** Topische anthelmintische Formulierung nach Anspruch 1 oder 2, umfassend Clorsulon und Ivermectin.

**7.** Topische anthelmintische Formulierung nach einem der vorhergehenden Ansprüche mit einer solchen Wirksamkeit, dass bei Anwendung auf die Haut eines Lebewesens, das durch F. hepatica infiziert ist, mindestens 90 % der reifen F. hepatica abgetötet werden.

**8.** Topische anthelmintische Formulierung nach Anspruch 5, worin die therapeutisch wirksame Menge von Clorsulon für Vieh mindestens 5 % (Gew./V) ist.

**9.** Topische anthelmintische Formulierung nach einem der vorhergehenden Ansprüche, worin der Träger eine polymere Spezies umfasst.

**10.** Topische anthelmintische Formulierung nach Anspruch 1 worin, die Anwendung mindestens 2 μg Benzolsulfonamid pro ml Blutplasma bereitstellt.

**11.** Topische anthelmintische Formulierung nach Anspruch 6 zur Anwendung bei Vieh, worin die Dosisrate gesteuert wird, um etwa 500 μg Ivermectin und 5 mg/kg Clorsulon bereitzustellen.

**12.** Topische anthelmintische Formulierung zur Anwendung bei Vieh, die als Aufgießformulierung vorliegt und besteht aus:

| | |
|---|---|
| Ivermectin 0,5 % (Gew./V) | Clorsulon 5 % (Gew./V) |
| Ethanol 30 % V/V | PEG200 10 % V/V |
| Crodamol Cap 20 % V/V | IPA (Isopropanol) auf 100 % V/V |
| Brillant Blue-Farbstoff 0,01 % (Gew./V) | Denatoniumbenzoat 0,001 % (Gew./V) |

**Revendications**

**1.** Formulation anthelminthique à usage topique comprenant, comme ingrédients actifs, une quantité thérapeutiquement efficace d'au moins un agent anthelminthique dérivé de *Streptomyces avermitilis,* avec une quantité thérapeutiquement efficace d'au moins un autre anthelminthique du type sulfonamide, dans un véhicule à base de solvants alcooliques convenant pour l'administration topique et l'apport des ingrédients actifs par voie transdermique, ledit véhicule comprenant au moins 30 % (en volume/volume) d'éthanol ainsi qu'une quantité d'isopropanol suffisante pour compléter à 100 % et facultativement des excipients et des adjuvants de formulation.

**2.** Formulation anthelminthique à usage topique selon la revendication 1, dans laquelle la formulation est présentée sous forme d'un produit à verser.

**3.** Formulation anthelminthique à usage topique selon la revendication 1, dans laquelle la formulation comprend une avermectine.

**4.** Formulation anthelminthique à usage topique selon la revendication 1, dans laquelle la formulation comprend de l'ivermectine.

**5.** Formulation anthelminthique à usage topique selon l'une quelconque des revendications précédentes, dans laquelle l'anthelminthique du type sulfonamide est le clorsulon.

**6.** Formulation anthelminthique à usage topique selon la revendication 1 ou la revendication 2, comprenant du clorsulon et de l'ivermectine.

**7.** Formulation anthelminthique à usage topique selon l'une quelconque des revendications précédentes, ayant une efficacité telle que, lorsqu'elle est appliquée à la peau d'un animal infecté par *F. hepatica,* au moins 90 % des *F. hepatica* matures sont tués.

8. Formulation anthelminthique à usage topique selon la revendication 5, dans laquelle la quantité thérapeutiquement efficace de clorsulon pour le bétail est d'au moins 5 % (en poids/volume).

9. Formulation anthelminthique à usage topique selon l'une quelconque des revendications précédentes, dans laquelle le véhicule comprend une espèce polymère.

10. Formulation anthelminthique à usage topique selon la revendication 1, qui fournit, à l'emploi, au moins 2 $\mu$g d'un benzènesulfonamide par ml de plasma sanguin.

11. Formulation anthelminthique à usage topique selon la revendication 6, à utiliser sur du bétail, dans laquelle le taux de dose est réglé pour fournir environ 500 $\mu$g d'ivermectine et 5 mg/kg de clorsulon.

12. Formulation anthelminthique à usage topique à utiliser sur du bétail, présentée sous forme d'un produit à verser et constituée de :

Ivermectine 0,5 % (en poids/volume)
Clorsulon 5,0 % (en poids/volume)
Éthanol 30 % en volume/volume
PEG200 10 % en volume/volume
Crodamol Cap 20 % en volume/volume
IPA (isopropanol) jusqu'à 100 % en volume/volume
Colorant Bleu Brillant 0,01 % (en poids/volume)
Benzoate de dénatonium 0,001 % (en poids/volume).

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030180350 A
- EP 0125004 A